# EUROPÄISCHE PATENTSCHRIFT

(11) **EP 1 260 243 B1**
(45) Veröffentlichungstag und Bekanntmachung des Hinweises auf die Patenterteilung: **23.11.2005**
(21) Anmeldenummer: 02007447.2
(22) Anmeldetag: 30.03.2002
(51) Int. Cl.: A61M 16/00, F04D 29/66

(54) **Vorrichtung zur Schalldämmung bei Beatmungsgeräten**
Noise dampening apparatus for a breathing device
Système pour l'insonorisation d'un appareil d'assistance respiratoire

(30) Priorität: 15.05.2001 DE 10123552
(43) Veröffentlichungstag der Anmeldung: 27.11.2002
(73) Patentinhaber: Weinmann Geräte für Medizin GmbH & Co. KG, 22525 Hamburg (DE)
(72) Erfinder: Franke, Stefan, 22547 Hamburg (DE); Timm, Thomas, 22415 Hamburg (DE)
(74) Vertreter: Klickow, Hans-Henning

(56) Entgegenhaltungen:
- EP-A- 0 545 003
- WO-A-99/22794
- DE-U- 20 013 392
- FR-A- 2 663 547

## Beschreibung

Die Erfindung betrifft eine Vorrichtung zur Schalldämmung bei der Beatmung eines Patienten, die als eine Dämpfungsbox ausgebildet ist, die einen von einem Gehäuse umschlossenen Innenraum aufweist, der mit mindestens einem Atemgaseinlaß und mit mindestens einem Atemgasauslaß versehen ist und der einen Aufnahmeraum für eine Gebläseeinheit mit Luftförderelement und Gebläsemotor aufweist sowie bei der die Gebläseeinheit von mindestens einer Feder innerhalb des Aufnahmeraumes gegen mindestens eine wandung des Aufnahmeraumes abgestützt ist.

Eine derartige Vorrichtung wird beispielsweise in der DE-OS 197 31 355 beschrieben. Der Innenraum der Dämpfungsbox wird dabei durch Strömungsleitelemente unterteilt, um einen im Hinblick auf die Geräuschdämmung optimierten Strömungsweg bereitzustellen. Gemäß dem Stand der Technik ist es üblich, in ein metallisches Gehäuse der Dämpfungsbox zur Verbesserung der Dämpfungswirkung Platten aus Schaumstoff einzukleben.

Die Vorrichtung wird typischerweise dafür eingesetzt, um die Ansauggeräusche der Gebläseeinheit sowie die vom Gebläsemotor und dem Luftförderelement erzeugten Geräusche zu reduzieren. Insbesondere im Hinblick auf die vom Gebläsemotor erzeugten Geräusche und Vibrationen können die bekannten Vorrichtungen noch nicht alle Anforderungen in optimaler Weise erfüllen

Aus der DE 200 13 392 U ist bereits eine vorrichtung zur Schalldämmung bekannt, die als eine Dämpfungsbox ausgebildet ist. Die Dämpfungsbox weist einen von einem Gehäuse umschlossenen Innenraum auf, der mit einem Atemgaseinlaß und einem Atemgasauslaß versehen ist. Im Aufnahmeraum ist eine Gebläseeinheit angeordnet und von einer Feder gegenüber einer Wandung abgestützt. Die Feder wird durch federnde Eigenschaften einer Schaumstoffschicht bereitgestellt.

Aus der FR-A-2 663 547 ist ebenfalls ein innerhalb einer Dämpfungsbox angeordneter Ventilator bekannt. Auch hier wird der ventilator auf Blöcken aus Schaumstoff gehaltert.

In der EP-A-0 545 033 wird eine Halterung für einen Elektromotor beschrieben. Der Elektromotor wird von gummielastischen Abstützungen in einer radialen Richtung gehaltert.

Aus der WO 99/22794 A ist ein Gebläse für ein Beatmungsgerät zur Durchführung der CPAP-Therapie bekannt. zur Geräuschdämpfung sind innerhalb eines Gehäuses angeordnete Strömungswege mit einer speziellen Kontur versehen.

Aufgabe der vorliegenden Erfindung ist es, eine Vorrichtung der einleitend genannten Art derart zu konstruieren, daß verbesserte Eigenschaften im Hinblick auf eine Dämpfung erreicht werden.

Diese Aufgabe wird erfindungsgemäß dadurch gelöst, daß die Feder einen Konturverlauf aufweist, der für die Gebläseeinheit einen Bewegungsfreiraum in drei räumlichen Dimensionen bereit stellt.

Gegenüber einer gemäß dem Stand der Technik bekannten Abstützung der Gebläseeinheit über Schaumstoffelemente bietet die Verwendung der Feder die Möglichkeit, eine verbesserte Körperschallentkopplung zu realisieren, die zu einer erheblichen Geräuschdämmung beiträgt. Darüber hinaus werden auch Vibrationen erheblich reduziert. Die Verwendung der Feder weist zusätzlich bei einem Einwirken von Stoßbelastungen den Vorteil auf, daß starke Bremskräfte oder Beschleunigungskräfte, die auf den Motor einwirken, vermieden werden. Insbesondere die Lager des Motors erweisen sich als gegenüber derartigen Kräften empfindlich.

Eine sehr kompakte Ausführungsform kann dadurch erreicht werden, daß die Feder als ein Federbügel ausgebildet ist.

Zur Bereitstellung von federnden Eigenschaften in mehreren räumlichen Richtungen wird vorgeschlagen, daß die Feder mehrfach gebogen ausgebildet ist.

Eine große Standfestigkeit wird dadurch unterstützt, daß die Feder eine rechteckförmige Grundfläche aufspannt.

Gute federnde Eigenschaften können insbesondere auch dadurch erreicht werden, daß die Feder aus einer Mehrzahl im wesentlichen linear verlaufender Segmente und einer Mehrzahl von Umbiegungen mit einem Biegewinkel von jeweils etwa 90° ausgebildet ist.

Zur Bereitstellung jeweils erforderlicher Federwege wird vorgeschlagen, daß die Feder einen Konturverlauf aufweist, der für die Gebläseeinheit einen Bewegungsfreiraum in drei räumlichen Dimensionen bereit stellt.

Zur Sicherstellung einer elektrischen Versorgung des Gebläsemotors auch bei der Durchführung von federnden Bewegungen ist vorgesehen, daß die Gebläseeinheit über flexible Anschlußleitungen elektrisch mit Energie versorgt ist.

Beschädigungen der elektrischen Anschlüsse bei der Durchführung von Federbewegungen können dadurch vermieden werden, daß die Länge der Anschlußleitungen an einen von der Feder bereitgestellten Federweg angepaßt ist.

Eine Kühlung des Gebläsemotors kann dadurch unterstützt werden, daß die Gebläseeinheit im Bereich eines Gehäusedeckels einen Gebläseeinlaß aufweist, der einen Gebläseinnenraum mit dem Aufnahmeraum verbindet.

Eine weitere Verbesserung der schalldämmenden Eigenschaften kann dadurch erreicht werden, daß der Aufnahmeraum mindestens bereichsweise mit einem schalldämmenden Material ausgekleidet ist.

Insbesondere ist daran gedacht, daß das schalldämmende Material als ein Schaumstoff ausgebildet ist.

Eine weitere Verbesserung der Dämpfungseigenschaften durch Bereitstellung von labyrinthartigen Strömungswegen kann dadurch erreicht werden, daß in einem Innenraum benachbart zum Aufnahmeraum mindestens ein Strömungsleitelement angeordnet ist.

Zur weiteren Verbesserung der Körperschallentkopplung wird vorgeschlagen, daß zwischen der Feder und der Wandung des Aufnahmeraumes ein schalldämmendes Material angeordnet ist.

Zur Bereitstellung einer ausreichenden Flexibilität bei der Ankopplung der Gebläseeinheit an die Strömungswege wird vorgeschlagen, daß ein Ausströmstutzen der Gebläseeinheit über ein elastisches Kopplungselement mit einem Auslaßkanal gekoppelt ist.

Eine weitere Verminderung der Geräuschemission kann dadurch erreicht werden, daß ein Lüfterrad der Gebläseeinheit räumlich versetzt zum Ausströmstutzen angeordnet ist.

Eine Verminderung von Strömungsgeräuschen innerhalb der Gebläseeinheit selbst kann dadurch unterstützt werden, daß ein Gebläseinnenraum mit gerundeten Innenraumkonturen versehen ist.

In der Zeichnung sind Ausführungsbeispiele der Erfindung schematisch dargestellt. Es zeigen:
- Fig. 1: eine Draufsicht auf ein Gehäuse der Vorrichtung mit abgenommenen Deckel,
- Fig. 2: einen Querschnitt gemäß Schnittlinie II-II in Fig. 1 nach einem Aufsetzen des Deckels,
- Fig. 3: eine Darstellung ähnlich zu Fig. 1 nach einem Einsetzen der Gebläseeinheit in den Aufnahmeraum,
- Fig. 4: einen Querschnitt gemäß Schnittlinie IV-IV in Fig. 3,
- Fig. 5: eine Ansicht gemäß Blickrichtung V in Fig. 3,
- Fig. 6: eine vergrößerte Darstellung der von einer bügelartigen Feder gehalterten Gebläseeinheit,
- Fig. 7: eine Ansicht gemäß Blickrichtung VII in Fig. 6,
- Fig. 8: eine Draufsicht auf die bügelartig ausgebildete Feder zur Halterung der Gebläseeinheit,
- Fig. 9: eine Darstellung gemäß Blickrichtung IX in Fig. 8,
- Fig. 10: eine Darstellung entsprechend Blickrichtung X in Fig. 8,
- Fig. 11: eine vergrößerte Darstellung der Gebläseeinheit und
- Fig. 12: eine Darstellung entsprechend Schnittstelle XII-XII in Fig. 11.

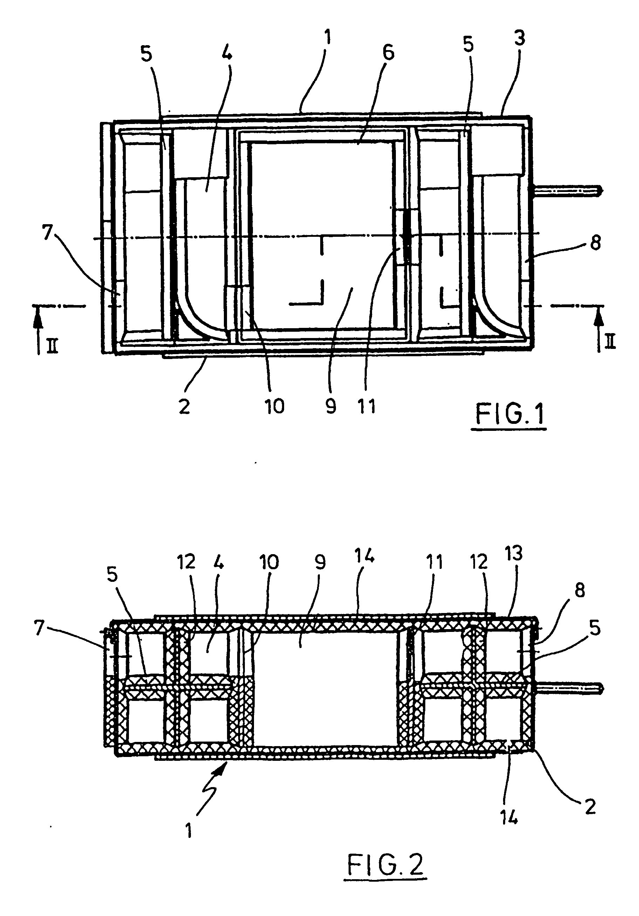

Figur 1 zeigt eine Draufsicht auf eine Dämpfungsbox (1), wobei exakt ein Gehäuseunterteil (2) dargestellt ist. Das Gehäuseunterteil (2) weist Wandungen (3) auf, die vorzugsweise aus Metall ausgebildet sind. Ein von der Dämpfungsbox (1) umschlossener Innenraum (4) kann beispielsweise quaderförmig begrenzt sein.

In das Gehäuseunterteil (2) sind zwei Strömungsleitelemente (5) sowie eine Feder (6) zur Halterung einer nicht dargestellten Gebläseeinheit eingesetzt.

Dem Innenraum (4) der Dämpfungsbox (1) wird Atemgas über einen Atemgaseinlaß (7) zugeführt. Das Atemgas verläßt die Dämpfungsbox (1) über einen Atemgasauslaß (8). Ein Teil des Innenraumes (4) ist als ein Aufnahmeraum (9) für die Feder (6) und die nicht dargestellte Gebläseeinheit ausgebildet. Der Aufnahmeraum (9) ist über einen Einlaßkanal (10) mit dem eingangsseitigen Strömungsleitelement (5) und über einen Auslaßkanal (11) mit dem ausgangsseitigen Strömungsleitelement (5) verbunden.

Aus der Querschnittdarstellung in Fig. 2 ist erkennbar, wie durch die Strömungsleitelemente (5) eine labyrinthartige Strömungsführung bereitgestellt wird. Insbesondere ist auch erkennbar, wie die Überleitung der Atemgasströmung in den Bereich des Aufnahmeraumes (9) erfolgt. Darüber hinaus ist zu erkennen, daß die Wandungen des Strömungsleitelementes (5) mit schalldämmendem Material (12) versehen sind. Das schalldämmende Material (12) kann auf die Wandungen aufgeklebt sein.

Ebenfalls ist aus Fig. 2 erkennbar, daß das Gehäuseunterteil (2) von einem Deckel (13) verschlossen ist und daß sowohl das Gehäuseunterteil (2) als auch der Deckel (13) mit schalldämmendem Material (14) ausgekleidet sind.

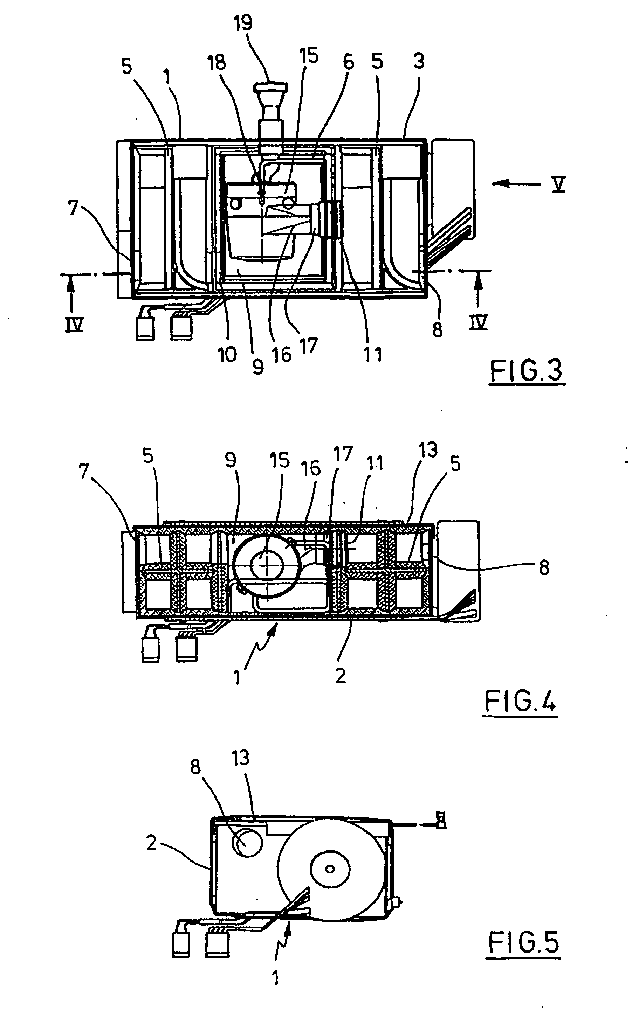

Fig. 3 zeigt die Anordnung gemäß Fig. 1 nach einem Einsetzen der Gebläseeinheit (15) in den Aufnahmeraum (9). Die Gebläseeinheit (15) ist über einen Ausströmstutzen (16) und ein elastisches Kopplungselement (17) mit dem Auslaßkanal (11) verbunden. Die Gebläseeinheit (15) wird von der Feder (6) gehaltert und wird über Anschlußleitungen (18) mit einem Kopplungselement (19) verbunden, das seinerseits vom Gehäuseunterteil (2) gehaltert ist.

Die Querschnittsdarstellung in Fig. 4 veranschaulicht, daß die Gebläseeinheit (15) von der bügelartig gebogenen Feder (6) innerhalb des Aufnahmeraumes gehaltert wird. Insbesondere ist erkennbar, daß durch die Biegung der Feder (6) ein ausreichender Bewegungsfreiraum für die Gebläseeinheit (15) innerhalb des Aufnahmeraumes (9) realisiert ist, um zum einen eine Körperschallentkopplung zu erreichen und darüber hinaus Ausgleichsbewegungen bei der Einwirkung von Stoßkräften bereitzustellen.

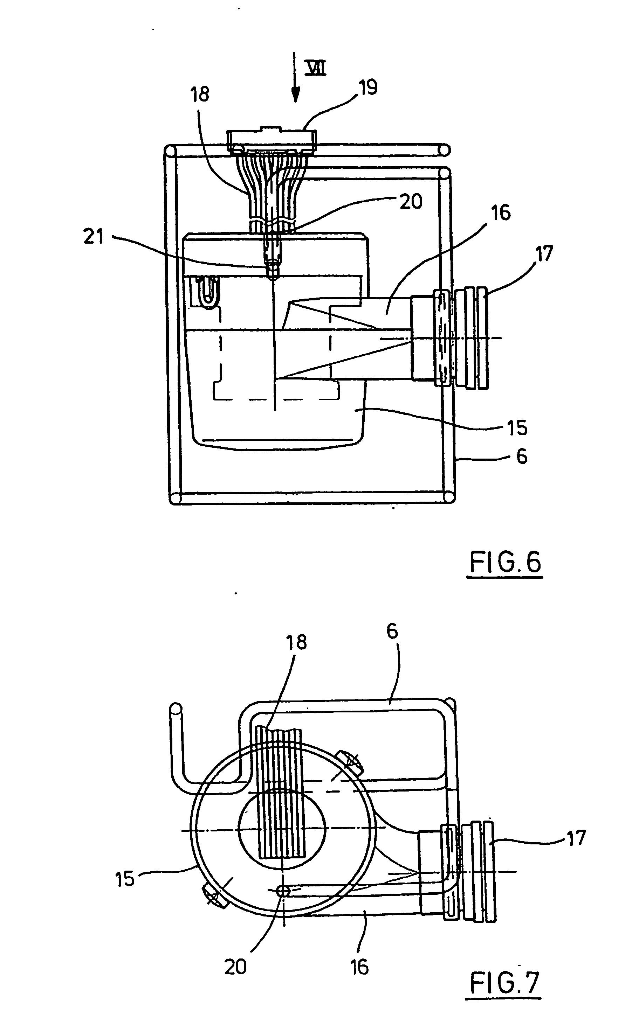

Die Seitenansicht in Fig. 5 veranschaulicht die kastenartige Gestaltung der Dämpfungsbox (1) sowie die geometrische Anordnung des Atemgasauslasses (8).

Fig. 6 veranschaulicht die Halterung der Gebläseeinheit (15) durch die Feder (6) in einer stärkeren Detailliertheit. Insbesondere ist erkennbar, daß die Feder (6) bei der dargestellten Ausführungsform aus einem gebogenen Federdraht besteht, bei dem jeweils im wesentlichen lineare Segmente durch Umbiegungen ineinander übergeleitet sind. Die Umbiegungen weisen jeweils einen Winkel im Bereich von 90° auf.

In einer Draufsicht spannt die Feder (6) eine rechteckförmige Grundfläche auf, und im Bereich der Umbiegungen werden jeweils Bewegungsmöglichkeiten bereitgestellt, die in der Gesamtheit dazu führen, daß die Gebläseeinheit (15) in allen drei räumlichen Dimensionen Ausgleichsbewegungen durchführen kann.

Bei der in Fig. 6 dargestellten Ausführungsform ragt ein Halterungsende (20) der Feder (6) in eine Halterungsausnehmung (21) der Gebläseeinheit (15) hinein. Grundsätzlich ist es aber auch möglich, die Gebläseeinheit (15) an mehr als einer Stelle federnd zu haltern.

Die Seitenansicht in Fig. 7 veranschaulicht ebenfalls, welche Bewegungsfreiräume für die Gebläseeinheit (15) durch die Feder (6) bereitgestellt werden. Darüber hinaus ist auch noch einmal der gebogene Verlauf der Feder (6) zu erkennen, der aus den linearen Segmenten sowie den Umbiegungen besteht.

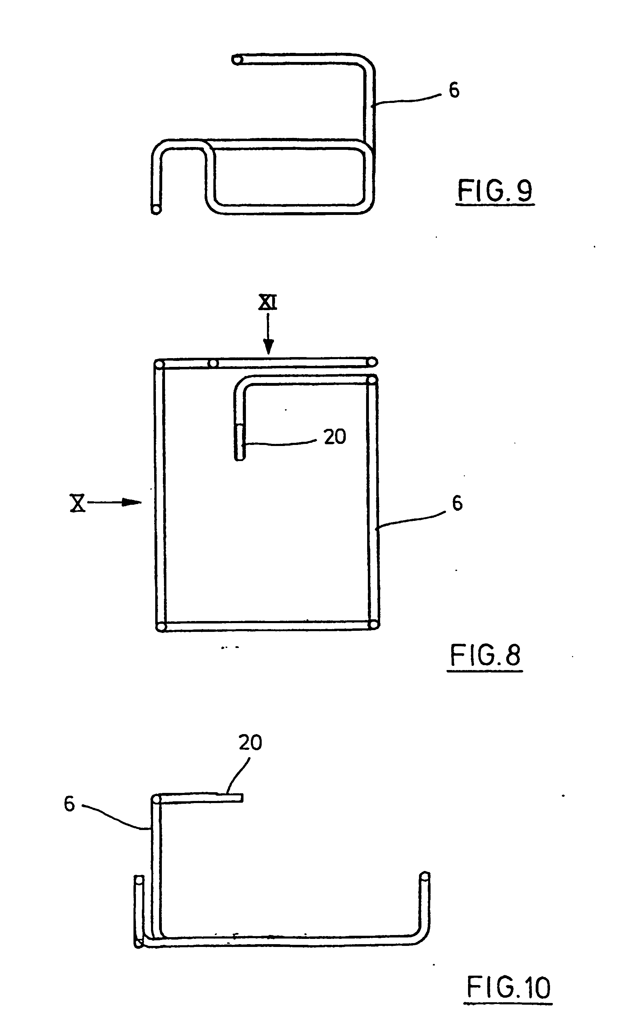

Fig. 8 zeigt die Feder (6) ohne gehalterte Gebläseeinheit (15) bei einer Orientierung gemäß Fig. 6. Der Konturverlauf der einzelnen Abschnitte der Feder (6) ist hierdurch besser zu erkennen.

Fig. 9 zeigt die Feder gemäß Fig. 8 in einer Seitenansicht. Es sind hier ebenfalls insbesondere die Vielzahl der linearen Segmente sowie der Umbiegungen erkennbar, die es erlauben, bei Verwendung nur einer Feder (6) Ausweichbewegungen der Gebläseeinheit (15) in drei räumlichen Dimensionen sowie die angestrebte Schallentkopplung zu realisieren.

Der geometrische Verlauf der einzelnen Bestandteile der Feder (6) wird durch die Seitenansicht in Fig. 10 nochmals weiter veranschaulicht. Es ist insbesondere auch erkennbar, daß im Bereich des Halterungsendes (20) eine Materialabflachung vorgesehen ist, um eine verdrehsichere Halterung der Gebläseeinheit (15) zu unterstützen.

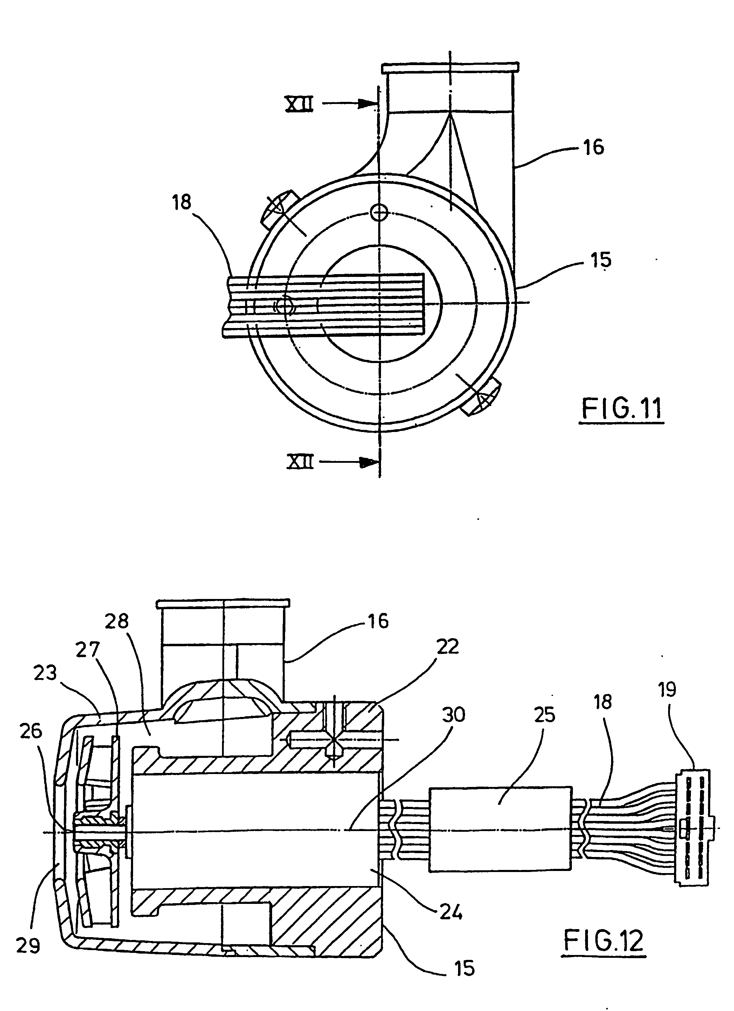

Fig. 11 zeigt die Gebläseeinheit (15) gemäß Fig. 7 in einer gedrehten räumlichen Orientierung und ohne die Feder (6).

Fig. 12 zeigt einen Längsschnitt durch die Gebläseeinheit (15) gemäß Schnittlinie XII-XII in Fig. 11. Es ist insbesondere erkennbar, daß die Gebläseeinheit (15) einen Gehäusesockel (22) und einen Gehäusedeckel (23) aufweist. Der Gehäusesockel (22) haltert einen Gebläsemotor (24), der über die Anschlußleitungen (18) elektrisch versorgt wird. Zur Vermeidung einer Beschädigung der Anschlußleitungen (16) bei der Herausführung aus der Dämpfungsbox (1) sind die Anschlußleitungen (18) abschnittsweise von einem Einfassungselement (25) umschlossen.

Der Gebläsemotor (24) trägt auf einer Motorwelle (26) ein Lüfterrad (27). Das Lüfterrad (27) rotiert in einem Gebläseinnenraum (28), der über einen Gebläseeinlaß (29) im Gehäusedeckel (23) mit einer Umgebung verbunden ist. In Richtung einer Gebläselängsachse (30) sind die Rotationsebene des Lüfterrades (27) und der Ausströmstutzen (16) versetzt zueinander angeordnet. Dies führt ebenfalls zu einer erhöhten Geräuschdämpfung. Eine zusätzliche Verminderung von Strömungsgeräuschen wird durch abgerundete Kanten mit ausreichend großen Krümmungsradien im Bereich aller Begrenzungen des Gebläseinnenraumes (28) erreicht.

Zwischen dem Lüfterrad (27) und dem Gehäusesockel (22) bzw. dem Gehäusedeckel (23) sind Abstände derart vorgegeben, daß bei einer Rückatmung eine flache Kennlinie erreicht wird. Dies führt dazu, daß bei einem Rückatmen durch das Gebläse hindurch lediglich ein geringer Druckanstieg verursacht wird. Auch hierdurch können Schallemissionen vermindert und ein erforderlicher Regelungsaufwand reduziert werden.

Der Gehäusesockel (22) stellt einen Massekörper bereit, der ebenfalls zu einer Verminderung der Schallemissionen des Gebläsemotors (24) beiträgt. Bei einer Veränderung der Drehzahl des Gebläsemotors (24) führt der Massekörper des Gehäusesockels (22) dazu, daß eine ausreichende Trägheitsmasse bereitsteht, um für einen ruhigen Lauf sowie eine stabile Einbaulage zu sorgen.

Bei einem Einbau der Feder (6) im Gehäuseunterteil (2) ist insbesondere auch daran gedacht, die Feder (6) nicht unmittelbar gegen die Wandungen (3) grenzen zu lassen, sondern auch im Bereich des Aufnahmeraumes (9) eine Auskleidung der Wandungen (3) mit schalldämmendem Material (14) vorzunehmen und hierdurch dieses schalldämmende Material (14) auch zwischen der Feder (6) und der Wandung (3) zu positionieren. Dies stellt eine weitere Maßnahme zur Erhöhung der Körperschallentkopplung dar.

Durch die Halterung der Gebläseeinheit (15) durch die Feder (6) innerhalb des Aufnahmeraumes (9) wird um die Gebläseeinheit (15) herum ein Freiraum bereitgestellt, der vom Atemgas vor einem Eintritt in den Gebläseeinlaß (29) durchströmt wird. Hierdurch wird eine sehr gute Kühlung der Gebläseeinheit (15) hervorgerufen. In Abhängigkeit von den jeweiligen Anwendungsanforderungen kann die Gebläseeinheit (15) sowohl einstufig als auch zweistufig realisiert werden.

## Patentansprüche

1. Vorrichtung zur Schalldämmung bei der Beatmung eines Patienten, die als eine Dämpfungsbox (1) ausgebildet ist, die einen von einem Gehäuse umschlossenen Innenraum (4) aufweist, der mit mindestens einem Atemgaseinlaß und mit mindestens einem Atemgasauslaß versehen ist und der einen Aufnahmeraum (9) für eine Gebläseeinheit (15) mit Luftförderelement und Gebläsemotor aufweist sowie bei der die Gebläseeinheit (15) von mindestens einer Feder (6) innerhalb des Aufnahmeraumes (9) gegen mindestens eine Wandung (3) des Aufnahmeraumes (9) abgestützt ist **dadurch gekennzeichnet, daß** die Feder einen Konturverlauf aufweist, der für die Gebläseeinheit (15) einen Bewegungsfreiraum in drei räumlichen Dimensionen bereitstellt.

2. Vorrichtung nach Anspruch 1, **dadurch gekennzeichnet, daß** die Feder (6) als ein Federbügel ausgebildet ist.

3. Vorrichtung nach Anspruch 1 oder 2, **dadurch gekennzeichnet, daß** die Feder (6) mehrfach gebogen ausgebildet ist.

4. Vorrichtung nach einem der Ansprüche 1 bis 3, **dadurch gekennzeichnet, daß** die Feder (6) eine rechteckförmige Grundfläche aufspannt.

5. Vorrichtung nach einem der Ansprüche 1 bis 4, **dadurch gekennzeichnet, daß** die Feder (6) aus einer Mehrzahl im wesentlichen linear verlaufender Segmente und einer Mehrzahl von Umbiegungen mit einem Biegewinkel von jeweils etwa 90° ausgebildet ist.

6. Vorrichtung nach einem der Ansprüche 1 bis 5, **dadurch gekennzeichnet, daß** die Gebläseeinheit (15) über flexible Anschlußleitungen (18) elektrisch mit Energie versorgt ist.

7. Vorrichtung nach Anspruch 6, **dadurch gekennzeichnet, daß** die Länge der Anschlußleitungen (18) an einen von der Feder (6) bereitgestellten Federweg angepaßt ist.

8. Vorrichtung nach einem der Ansprüche 1 bis 7, **dadurch gekennzeichnet, daß** die Gebläseeinheit (15) im Bereich eines Gehäusedeckels (23) einen seinnenraum (28) mit dem Aufnahmeraum (9) verbindet.

9. Vorrichtung nach einem der Ansprüche 1 bis 8, **dadurch gekennzeichnet, daß** der Aufnahmeraum (9) mindestens bereichsweise mit einem schalldämmenden Material (14) ausgekleidet ist.

10. Vorrichtung nach Anspruch 9, **dadurch gekennzeichnet, daß** das schalldämmende Material als ein Schaumstoff ausgebildet ist.

11. Vorrichtung nach einem der Ansprüche 1 bis 10, **dadurch gekennzeichnet, daß** in einem Innenraum (4) benachbart zum Aufnahmeraum (9) mindestens ein Strömungsleitelement (5) angeordnet ist.

12. Vorrichtung nach einem der Ansprüche 1 bis 11, **dadurch gekennzeichnet, daß** zwischen der Feder (6) und der Wandung (3) des Aufnahmeraumes (9) ein schalldämmendes Material (14) angeordnet ist.

13. Vorrichtung nach einem der Ansprüche 1 bis 12, **dadurch gekennzeichnet, daß** ein Ausströmstutzen (16) der Gebläseeinheit (15) über ein elastisches Kopplungselement (17) mit einem Auslaßkanal (11) gekoppelt ist.

14. Vorrichtung nach einem der Ansprüche 1 bis 13, **dadurch gekennzeichnet, daß** ein Lüfterrad (27) der Gebläseeinheit (15) räumlich versetzt zum Ausströmstutzen (16) angeordnet ist.

15. Vorrichtung nach einem der Ansprüche 1 bis 14, **dadurch gekennzeichnet, daß** ein Gebläseinnenraum (28) mit gerundeten Innenraumkonturen versehen ist.

## Claims

1. A device for reducing noise when ventilating a patient, which is constructed as a damping box (1) which has an interior (4) which is surrounded by a housing and is provided with at least one breathing gas inlet and with at least one breathing gas outlet and which has a receiving chamber (9) for a ventilator unit (15) having an air delivery element and a ventilator motor and in which the ventilator unit (15) is supported against at least one wall (3) of the receiving chamber (9) by at least one spring (6) inside the receiving chamber (9), **characterised in that** the spring has a contour progression which provides a free movement space in three spatial dimensions for the ventilator unit (15).

2. A device according to Claim 1, **characterised in that** the spring (6) is constructed as a spring clip.

3. A device according to Claim 1 or 2, **characterised in that** the spring (6) is constructed with a plurality of bends.

4. A device according to one of Claims 1 to 3, **characterised in that** the spring (6) spans a rectangular base area.

5. A device according to one of Claims 1 to 4, **characterised in that** the spring (6) is constructed from a plurality of substantially linearly extending segments and a plurality of bends, each having a bend angle of approximately 90°.

6. A device according to one of Claims 1 to 5, **characterised in that** the ventilator unit (15) is supplied with electrical energy by way of flexible connecting lines (18).

7. A device according to Claim 6, **characterised in that** the length of the connecting lines (18) is matched to a spring travel produced by the spring (6).

8. A device according to one of Claims 1 to 7, **characterised in that** the ventilator unit (15) connects a housing interior (28) to the receiving chamber (9) in the region of a housing cover (23).

9. A device according to one of Claims 1 to 8, **characterised in that** at least some regions of the receiving chamber (9) are lined with a noise reducing material (14) .

10. A device according to Claim 9, **characterised in that** the noise reducing material is constructed as a foam material.

11. A device according to one of Claims 1 to 10, **characterised in that** at least one flow conducting element (5) is arranged in an interior space (4) adjacent to the receiving chamber (9).

12. A device according to one of Claims 1 to 11, **characterised in that** a noise reducing material (14) is arranged between the spring (6) and the wall (3) of the receiving chamber (9).

13. A device according to one of Claims 1 to 12, **characterised in that** a discharge nozzle (16) of the ventilator unit (15) is coupled to an outlet channel (11) by way of an elastic coupling element (17).

14. A device according to one of Claims 1 to 13, **characterised in that** a fan wheel (27) of the ventilator unit (15) is arranged spatially offset from the discharge nozzle (16).

15. A device according to one of Claims 1 to 14, **characterised in that** a ventilator interior (28) is provided with rounded interior contours.

## Revendications

1. Dispositif d'insonorisation pour l'assistance respiratoire d'un patient, qui est réalisé sous la forme d'un caisson d'insonorisation (1), qui présente une chambre interne (4) entourée d'un boîtier, qui est pourvue d'au moins une admission d'air respiratoire et d'au moins une évacuation d'air respiratoire, et qui présente une chambre (9) de réception pour un système de soufflerie (15) avec un organe de déplacement d'air et un moteur de soufflerie, et dans lequel le système de soufflerie (15) est maintenu par au moins un ressort (6) à l'intérieur de la chambre de réception (9), contre au moins une paroi (3) de la chambre de réception (9), **caractérisé en ce que** le ressort présente un contour qui assure au système de soufflerie (15) un espace de mouvement libre dans trois dimensions spatiales.

2. Dispositif selon la revendication 1, **caractérisé en ce que** le ressort (6) est réalisé sous la forme d'un ressort en étrier.

3. Dispositif selon la revendication 1 ou 2, **caractérisé en ce que** le ressort (6) est plié plusieurs fois.

4. Dispositif selon l'une des revendications 1 à 3, **caractérisé en ce que** le ressort (6) délimite une surface de base rectangulaire.

5. Dispositif selon l'une des revendications 1 à 4, **caractérisé en ce que** le ressort (6) est réalisé à partir de plusieurs segments s'étendant sensiblement en ligne droite, et de plusieurs courbures avec un angle de courbure de chaque fois environ 90°.

6. Dispositif selon l'une des revendications 1 à 5, **caractérisé en ce que**
le système de soufflerie (15) est alimenté en énergie électrique par des lignes de connexion (18) flexibles.

7. Dispositif selon la revendication 6, **caractérisé en ce que** la longueur des lignes de connexion (18) est adaptée à un déplacement élastique assuré par le ressort (6).

8. Dispositif selon l'une des revendications 1 à 7, **caractérisé en ce que** le système de soufflerie (15) relie une chambre interne de soufflerie (28) avec la chambre de réception (9), dans la région d'un couvercle de boîtier (23).

9. Dispositif selon l'une des revendications 1 à 8, **caractérisé en ce que** la chambre de réception (9) est recouverte au moins en partie d'un matériau d'insonorisation (14).

10. Dispositif selon la revendication 9, **caractérisé en ce que** le matériau d'insonorisation est réalisé sous la forme d'une mousse.

11. Dispositif selon l'une des revendications 1 à 10, **caractérisé en ce qu'**au moins un organe (5) de guidage du flux est agencé dans une chambre interne (4), au voisinage de la chambre de réception (9).

12. Dispositif selon l'une des revendications 1 à 11, **caractérisé en ce qu'**un matériau d'insonorisation (14) est agencé entre le ressort (6) et la paroi (3) de la chambre de réception (9).

13. Dispositif selon l'une des revendications 1 à 12, **caractérisé en ce qu'**un embout d'évacuation (16) du système de soufflerie (15) est relié à un canal d'évacuation (11) par l'intermédiaire d'un organe d'accouplement élastique (17).

14. Dispositif selon l'une des revendications 1 à 13, **caractérisé en ce qu'**une roue de soufflerie (27) du système de soufflerie (15) est disposée décalée dans l'espace par rapport à l'embout d'évacuation (16).

15. Dispositif selon l'une des revendications 1 à 14, **caractérisé en ce qu'**une chambre interne de soufflerie (28) est pourvue de bords arrondis de chambre interne.
